# EUROPEAN PATENT SPECIFICATION

(11) **EP 3 661 459 B1**
(45) Date of publication and mention of the grant of the patent: **02.10.2024**
(21) Application number: 18842143.2
(22) Date of filing: 03.08.2018
(51) Int. Cl.: A61F 2/24, B29C 41/02

(54) **METHODS RELATING TO THE MANUFACTURE OF PROSTHETIC VALVES**
VERFAHREN IM ZUSAMMENHANG MIT DER HERSTELLUNG VON HERZKLAPPENPROTHESEN
PROCÉDÉS SE RAPPORTANT À LA FABRICATION DE VALVULES PROTHÉTIQUES

(30) Priority: 04.08.2017 US 201762541575 P; 17.08.2017 US 201762547034 P
(43) Date of publication of application: 10.06.2020
(73) Proprietor: Foldax, Inc., Salt Lake City, Utah 84103 (US)
(72) Inventor: BEITH, Jason G., Santa Ana California 92706 (US); LOMBARDI, Fabien N., Salt Lake City Utah 84102 (US)
(74) Representative: Booth, Catherine Louise
(86) International application number: PCT/US2018/045202
(87) International publication number: WO 2019/028374

(56) References cited:
- WO-A1-2013/142879
- US-A- 3 550 206
- US-A- 3 964 433
- US-A1- 2007 027 535
- US-A1- 2014 012 371
- US-A1- 2014 167 308
- US-A1- 2016 067 038
- US-A1- 2016 214 303
- US-A1- 2016 296 324
- US-A1- 2017 071 735

## Description

### FIELD

The subject matter described herein relates generally to improved replacement valves, and more particularly to improved techniques for the manufacturer and manufacturability of prosthetic valves, such as prosthetic heart valves having artificial polymeric leaflets.

### BACKGROUND

The human heart has a number of valves for maintaining the flow of blood through the body in the proper direction. The major valves of the heart are the atrioventricular (AV) valves, including the bicuspid (mitral) and the tricuspid valves, and the semilunar valves, including the aortic and the pulmonary valves. When healthy, each of these valves operates in a similar manner. The valve translates between an open state (that permits the flow of blood) and a closed state (that prevents the flow of blood) in response to pressure differentials that arise on opposite sides of the valve.

A patient's health can be placed at serious risk if any of these valves begin to malfunction. Although the malfunction can be due to a variety of reasons, it typically results in either a blood flow restricting stenosis or a regurgitation, where blood is permitted to flow in the wrong direction. If the deficiency is severe, then the heart valve may require replacement.

Substantial effort has been invested in the development of replacement heart valves, most notably replacement aortic and mitral valves. Replacement valves can be implanted percutaneously by way of a transfemorally or transapically introduced catheter, or can be implanted directly through open heart surgery. The replacement valves typically include an arrangement of valve leaflets that are fabricated from porcine tissue. These tissue leaflets are highly distensible or stretchable and manufacture of the valves involves manually sewing the leaflets to a support.

US 2016/067038 relates to a compressible prosthetic heart valve having a stent with three leaflets a ring-like body defining three arch-shaped elements including an arc and a pair of opposing haunches and three commissural posts formed by the haunches and adjacent elements. The leaflets each have an attachment edge and a free edge with a belly extending therebetween. The leaflets are movable between a coapted condition, in which the free edges abut and prevent fluid flow through the valve, and an open condition, in which fluid flow through the valve is permitted. The leaflets are made from a polymeric material and are created on a mold to which polymer is applied. This document separately discloses temperature and humidity parameters for spraying polymer directly onto a mold and temperature parameters for dipping a mold into polymer.

WO 2013/142879 discusses a single-layer tissue sheet having a puncture strength of 2 kgf to 6 kgf. A heart valve may be made from one or more leaflets formed from a single-layer tissue sheet. A method of making a tissue sheet having a puncture strength of 2 kgf to 5 kgf is described having very long culture times, such as in excess of 20 weeks. Valves that comprise one or more leaflets made from the ultra-strong tissue sheets may be delivered via trans-catheter aortic valve implantation. This document does not disclose any particular structures or manufacturing parameters for forming structural or leaflet materials from polymers.

US 2014/012371 relates to a collapsible stent for an implantable prosthetic valve incorporates leaflet-supporting members having relatively large surface area in comparison to other members defining the remainder of the stent. The leaflet-supporting members may have openings extending through them. The valve leaflets may be attached to the stent so that an attached edge of each leaflet extends along the leaflet-supporting members and is attached to the members by polymer integral with the leaflet overlying the leaflet supporting members and extending into the openings of the leaflet supporting members. The valve structure may be formulated from a flowing polymer that is then cured. This document does not disclose any particular structures or manufacturing parameters for forming the leaflets or leaflet-supporting members.

US 2007/027535 describes a mandrel used to form a valve leaflet and support structure and describes a conventional housing and polymer solvent removal conditions traditionally used with polymer coating methods. In this document, the polymer applied to the valve mandrel, frame or assemble is maintained in a heat chamber to dry off the solvent.

Other replacement valves have artificial polymeric leaflets integrally formed to a support structure. While these artificial leaflet valves have demonstrated promise, the techniques for manufacturing such artificial leaflets and valves are in their early stages and are not suitable for larger scale manufacturing.

For these and other reasons, needs exist for improved systems, devices, and methods for manufacturing prosthetic valves with artificial leaflets.

### SUMMARY

The present invention is defined in the appended claims.

Provided herein are a number of example embodiments of systems, devices, and methods for manufacturing or use in manufacturing a prosthetic heart valve. Many of these embodiments can utilize an environmental humidity chamber (EHC) during a phase of partially curing liquid polymer that will, after further processing, form leaflets on a valve frame. The EHC can be part of a multiple stage manufacturing process and, in some embodiments, the multiple stages can include applying the liquid polymer to the valve frame and a leaflet formation structure, then partially curing the liquid polymer in the EHC, and then removing the valve frame and leaflet formation structure and subjecting the partially cured polymer to another stage of curing. Variations to this process and additional steps can be implemented as well.

Also provided herein are a number of example embodiments of systems, devices, and methods for utilizing an identifier applied to a valve frame during a manufacturing process. The identifier can represent information about the valve or valve frame, and can be read using one of a number of various energy spectrums. In some embodiments, the identifier is an optically readable bar code or QR code. In some embodiments, the identifier includes a serial number or other unique identifier of the valve frame, a type of the valve or valve frame, and/or a size of the valve or valve frame. The identifier can be applied to the valve frame prior to the application of one or more coatings, and can remain readable through the coatings. The identifier can be used to maintain traceability of the valve throughout a manufacturing process and after. The identifier can be used to select the appropriate software for manufacturing the valve at various stages of the manufacturing process.

Other systems, devices, methods, features and advantages of the subject matter described herein will be or will become apparent to one with skill in the art upon examination of the following figures and detailed description. It is intended that all such additional systems, methods, features and advantages be included within this description, be within the scope of the subject matter described herein. In no way should the features of the example embodiments be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is a perspective view depicting an example embodiment of an artificial valve.
FIG. 2A is a photograph depicting an example embodiment of a valve frame.
FIG. 2B is a photograph depicting an example embodiment of a leaflet formation structure.
FIGs. 3A-3B are flow diagrams depicting example embodiments of methods of manufacturing.
FIGs. 4A-4B are block diagrams depicting example embodiments of a manufacturing facility.
FIGs. 5A-5B are photographs depicting a front of an example embodiment of an EHC.
FIG. 5C is a photograph depicting a side of an example embodiment of an EHC.
FIGs. 5D-5E are photographs depicting a top down view of an example embodiment of an EHC.
FIGs. 6-7 are photographs depicting an example embodiment of a valve frame in a marking fixture.
FIG. 8 is a photograph depicting an example embodiment of a valve frame with an identifier.
FIG. 9 is a photograph depicting an example embodiment of a valve frame identifier being read by a reader device.
FIG. 10A is a block diagram depicting an example embodiment of a devices and systems used in reading the identifier and utilizing the information represented thereby.
FIG. 10B is a flow diagram depicting an example embodiment of a method of using an identifier in a manufacturing process.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

Before the present subject matter is described in detail, it is to be understood that this disclosure is not limited to the particular embodiments described, as such may, of course, vary. It is also to be understood that the terminology used herein is for the purpose of describing particular embodiments only and is not intended to be limiting.

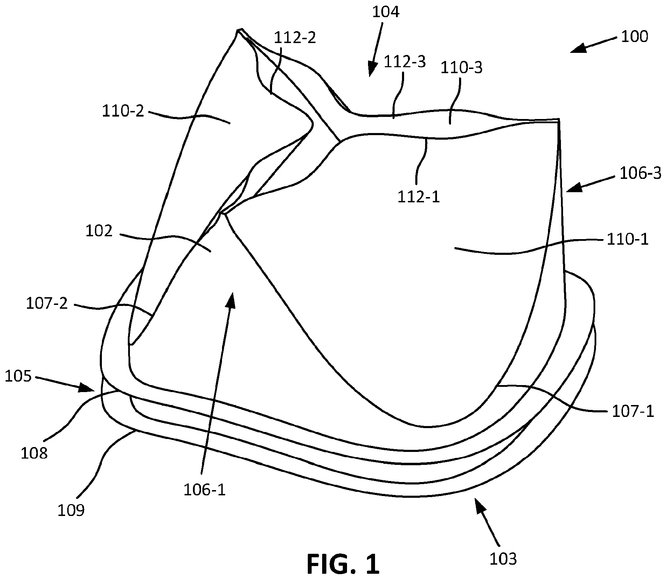

The example embodiments described herein relate to improved techniques for the manufacture and manufacturability of prosthetic valves, such as prosthetic heart valves having a support structure or frame coupled with two or more artificial polymeric leaflets. FIG. 1 is a perspective view depicting an example of a prosthetic valve 100 having frame 102 and three valve leaflets 110-1, 110-2, and 110-3. Valve 100 is configured to allow blood to flow from an upstream end 103 to a downstream end 104. Frame 102 has an annular base portion 105 that can have a planar or flat upstream terminus (not shown) or a curved or scalloped upstream terminus as shown here. Frame 102 also includes three extensions 106 that project from annular base portion 105 towards downstream end 104.

The downstream edges of extensions 106 have curved interfaces 107, which are the locations where support structure 102 meets the operable base of leaflets 110. Leaflets 110 each have a free edge 112, and leaflets 110 are free to move with respect to support structure 102 between open and closed positions for permitting and preventing, respectively, the flow of blood through valve 100. Each of leaflets 110 can be discrete from the others (as shown here) or can be portions of one unitary (monolithic) leaflet body. Leaflets 110 are integrally formed on this frame 102, such as through a casting (e.g., dip casting) or molding process.

Annular base portion 105 also includes flanges 108 and 109 between which a sewing cuff (not shown) can be placed. In other valves 100, only a single flange 108 may be present, or the flanges 108 and 109 can be omitted altogether. Examples of valves with sewing cuffs are described in U.S. Patent No. 6,716,244, U.S. Patent No. 6,755,857, U.S. Patent No. 7,641,687, and U.S. Patent Application Publication No. 2003/0023302. Other examples of prosthetic heart valves are described, for instance, in International Patent Application Publication No. WO 2015/171190, and U.S. Patent Publ. No. 2018/0116794A1.

Frame 102 can be fabricated from one or more materials (e.g., a core structure of one material with a coating of the same or another material). The materials are preferably polymeric materials such as polyether ether ketones (PEEK), polyurethanes, a polyetherimides (PEI) such as ULTEM, any of the materials used to form leaflets 110, and others. Leaflets 110 are also preferably fabricated from polymeric materials, including any biostable polyurethanes and polyurethane compositions (e.g., polysiloxane-containing polyurethanes, etc.) known in the art. Examples of polyurethane-containing leaflets are described in U.S. Patent No. 6,984,700, U.S. Patent No. 7,262,260, U.S. Patent No. 7,365,134, U.S. Patent Publ. No. 2017/0119923 ("Polyurethane/urea Compositions"), and Yilgor et al., "Silicone containing copolymers: Synthesis, properties and applications," Prog. Polym. Sci. (2013). Materials that approach ideal isotropic non-creeping characteristics are particularly suitable for use in many embodiments.

### Example Embodiments of Prosthetic Valve Manufacturing with Environmental Humidity Chambers

Numerous embodiments of systems, devices, and methods of manufacturing valves 100 having artificial polymeric leaflets 110 are described herein. Valves 100 are manufactured in a fashion that integrates leaflets 110 directly with frame 102 in a seamless manner, such as by coating a mandrel holding frame 102 with the polymer in liquid form. Thus, the same step of forming the leaflets also couples them to frame 102. The manufacturing embodiments will be described herein in the context of a dip casting or dipping process used to form the leaflets, however those of ordinary skill in the art will recognize that other formation processes (e.g., molding) can be used as well.

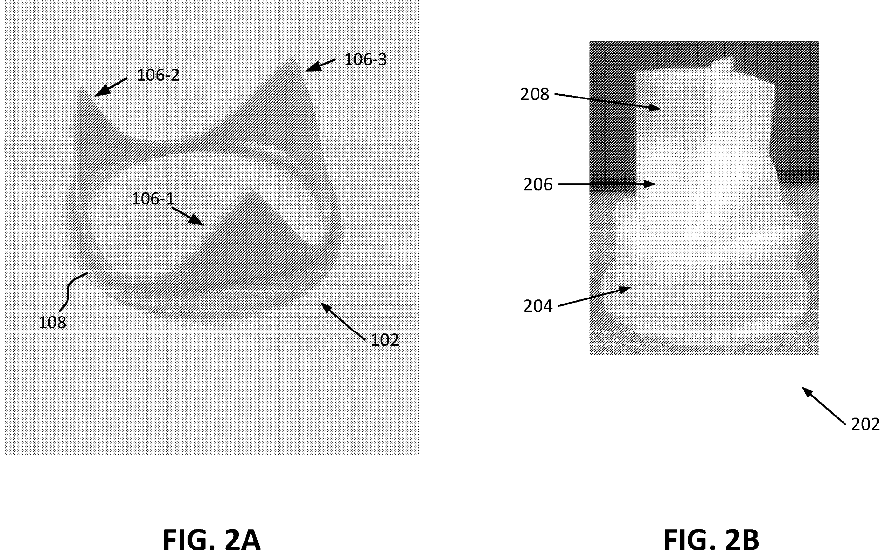

FIG. 2A is a photograph depicting an example of a frame 102, with a single flange 108, prior to formation of leaflets 110. FIG. 2B is a photograph depicting an example of a valve former or formation structure, also referred to as a mandrel 202, suitable for use in a dip casting method. In this example, mandrel 202 is sized to receive frame 102 at middle (or frame) portion 206, which has an exterior surface dimensioned to match the interior surface of frame 102, and also dimensioned to allow formation of leaflets thereon. The dimensions of middle portion 206 are varied depending on the size of frame 102 and the size (e.g., 17mm, 19mm, 21mm, etc.) and type of valve (e.g., aortic, mitral, or other) being manufactured. Mandrel 202 has a base portion 204 that can be grasped by manufacturing equipment during the manufacturing process. Base portion 204 can thus have uniform dimensions that are the same regardless of what size valve is being manufactured. During a dipping process mandrel 202 would be inverted from the orientation shown in FIG. 2B, and a tip portion 208 (which would thus be located at bottom) of mandrel 202 can be present to guide polymer runoff after frame 102 and mandrel 202 are dipped into a container of liquid polymer. 300

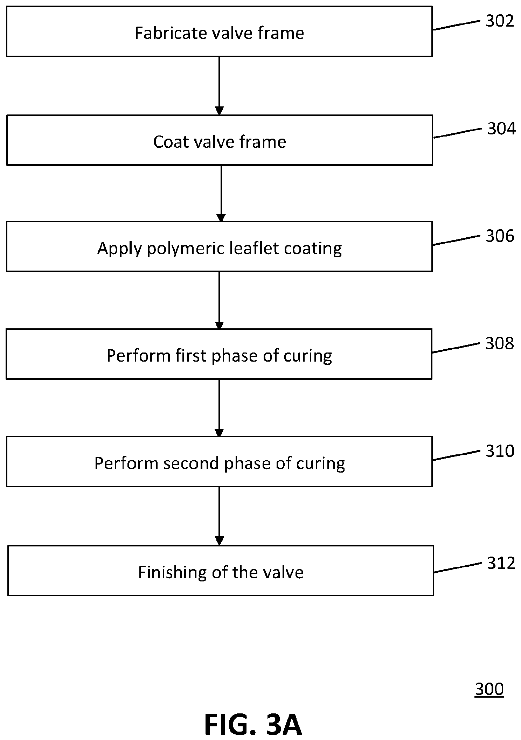

FIG. 3A is a flow diagram depicting an example embodiment of a method 300 of manufacturing a prosthetic valve 100. At 302, a support structure or frame 102 can be fabricated from a suitable material such as those described herein. This can be done, e.g., by machining (e.g., 3D printing), molding (e.g., injection molding), or otherwise. Then frame 102 can optionally be coated at 304. A coating step 304 can be advantageous if, e.g., frame 102 is fabricated from a first material (e.g., PEEK) different than the polymeric material from which the leaflets are fabricated. In that case it may be desirable to form the leaflets to frame 102 only after frame 102 has been pre-coated by the leaflet polymer to provide for greater cohesion. The frame can be coated by first dipping the core frame in the leaflet polymer having a first viscosity. This is preferably without mandrel 202 to avoid formation of leaflets. If done with mandrel 202, the resulting leaflets are removed. In other embodiments frame 102 can be coated in other manners, such as by spraying or brushing the coating material thereon. If frame 102 undergoes coating step 304 then this is preferably followed by a curing step that allows the coating to dry. The coating forms an undercoat on which the leaflets can be formed.

The leaflets can then be formed at step(s) 306. As stated herein, leaflet formation can occur in multiple ways and a dip casting process will be described as an example. Frame 102 can first be placed over mandrel 202 (if not already). Mandrel 202 (with frame 102 thereon) can then be inserted into a reservoir having the leaflet material in a liquid state (e.g., as a solution including solvent). Mandrel 202 is preferably dipped such that the solution envelops tip portion 208 and middle portion 206 of mandrel 202, including envelopment of frame 102 thereon. Mandrel 202 can be submersed further such that some or all of base portion 204 is covered as well. This dipping process preferably takes place under a relatively high temperature and high humidity. Although the methods disclosed herein are not limited to such, in some example embodiments, the relative humidity (RH) can be in the range of 20-80% and the temperature can be in the range of 20-50 degrees C.

Upon removal from the reservoir, frame 102 and mandrel 202 (including the leaflet casting surfaces) will be covered in a layer of liquid polymer and this layer can drip or runoff along portion 208 of mandrel 202. The dipping step 306 can be performed only once to arrive at the fully formed (but unfinished) valve 100, or can be performed multiple times (e.g., two times, three times, or as many times as desired). If multiple dipping steps are performed, then the dipping can occur into a reservoir with polymer having the same or a different viscosity.

After dipping is complete, mandrel 202 and valve 100 can be subjected to a first phase of curing at step 308, followed by a second phase of curing 310. The first phase of curing 308 can be a relatively short cure lasting a few minutes, and the second phase of curing 310 can be a relatively longer cure lasting several hours. The first phase of curing 308 can be performed within an environmental humidity chamber (EHC) that will be described in greater detail herein. Although purposes for performing the first phase of curing can vary, in many cases this first phase 308 is performed at process conditions that cause the liquid polymer to reach a viscosity or level of dryness sufficient to substantially slow or stop runoff or dripping, sometimes referred to as causing the leaflets to seize. The level of runoff impacts the thickness of the resulting leaflets 110, and thus their performance characteristics, making it desirable to maintain a high degree of control during manufacturing.

After first phase 308, excessive seized polymer can be trimmed if needed and valve 100 can be subjected to a second phase of curing 310. The second phase 310 can take place in a different chamber, such as an oven, and can cause the seized polymer to complete the drying process.

After curing step 310, valve 100 can be subjected to one or more finishing processes at 312, such as one or more of removal of mandrel 202, trimming, sewing cuff attachment, inspection, packaging, or the like. For example, leaflets 110 (and optionally frame 102) can be trimmed and/or otherwise finished to achieve accurate and precise edges and surface smoothness. This can occur, for example, through laser cutting, ultrasonic trimming, a water knife, a mechanical clam shell cutter, and the like. In some embodiments, valve 100 is trimmed while on mandrel 202 by cutting through the leaflet polymer and mandrel at the intersection between middle portion 206 and tip portion 208 of mandrel 202. A sewing cuff can be coupled with valve 100 (using any flange if present) if desired. Valve 100 can be subjected to a visual inspection and/or measurement. Valve 100 can then be packaged in the desired sterile container.

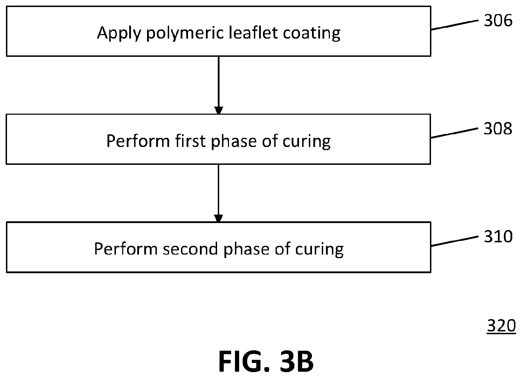

FIG. 3B is a flow diagram depicting an example embodiment of method 320 of forming leaflets 110 for a prosthetic valve 100. Here, method 320 includes the steps of dipping 306, first phase curing 308, and second phase curing 310, substantially as described with respect to the embodiment of FIG. 3A. The remaining steps of method 300, while they can be included, are optional to method 320.

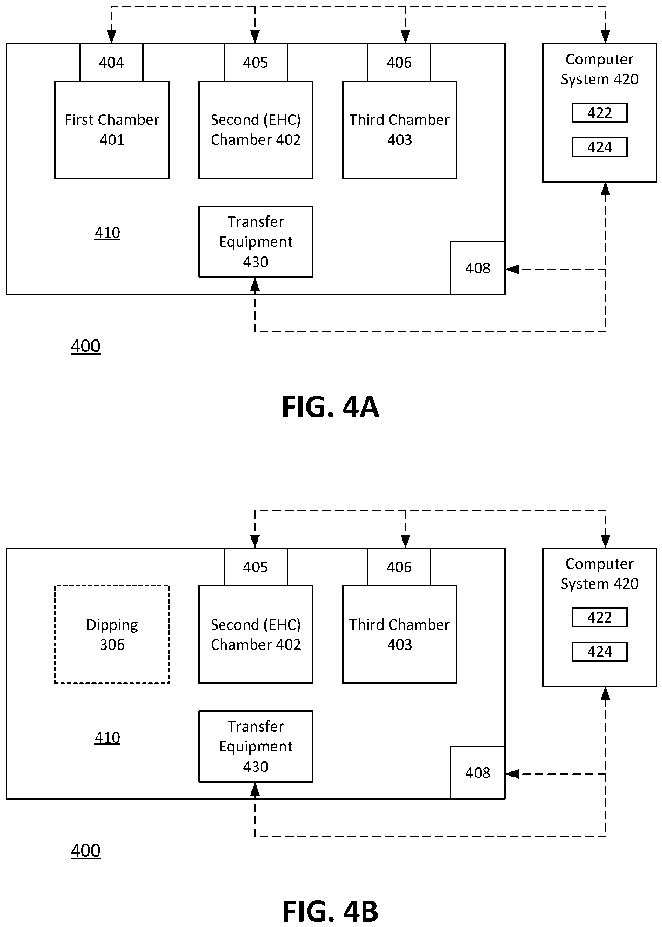

FIGs. 4A and 4B are block diagrams depicting example embodiments of manufacturing facilities 400 for valve 100. Facility 400 is preferably configured as a sealable clean environment with an internal environment 410 that is controlled with filtered airflow passageways entering facility 400 to limit or prevent particle contamination. The temperature, humidity, ambient pressure, and composition of the ambient gas (e.g., oxygen level, nitrogen level, etc.) within environment 410 can also be controlled and maintained at a desired level by environment control equipment 408. A computer system 420 having processing circuitry 422 executing control software stored in a non-transitory memory 424 can be communicatively coupled with control equipment 408 and can exercise control over equipment 408, and thus environment 410. Processing circuitry 422 can be one or more processors, controllers, programable logic controllers (PLCs), a combination thereof, and the like.

Facility 400 can include one or more internal chambers in which certain steps of valve manufacturing can be performed. The size of facility 400 can vary, and in some embodiments is only 1-3 meters in width by 1-3 meters in length. In the embodiment of FIG. 4A, facility 400 includes a first chamber 401 for performing dipping step 306, a second chamber 402 for performing first phase curing step 308, and a third chamber 403 for performing second phase curing step 310. Other chambers can be included for performance of other manufacturing steps if desired.

Each chamber 401-403 has its own internal environment that can be controlled independently of the environments within the other chambers and within the ambient space 410 of facility 400. Control of the internal environment of each chamber 401-403 can include control of temperature, humidity, composition of the internal gas, ambient gas pressure, and the like. Control of the internal environment of each chamber 401-403 can be performed automatically by software executed by processing circuitry 422 of master computer system 420, that is communicatively coupled with environmental control equipment 404-406 of each chamber 401-403, respectively. In the embodiment of FIG. 4B, separate chambers 402 and 403 are present for curing steps 308 and 310, but dipping step 306 is performed in the ambient environment 410 of facility 400. In some embodiments, dipping can be performed within EHC 402.

In the embodiments of FIGs. 4A and 4B, automated equipment 430 (e.g., robotic equipment) can be included for transferring frame 102 (with or without leaflets 110) from each location or chamber 401-403 to the next. Automated equipment 430 can also be communicatively coupled with and under the control of computer system 420.

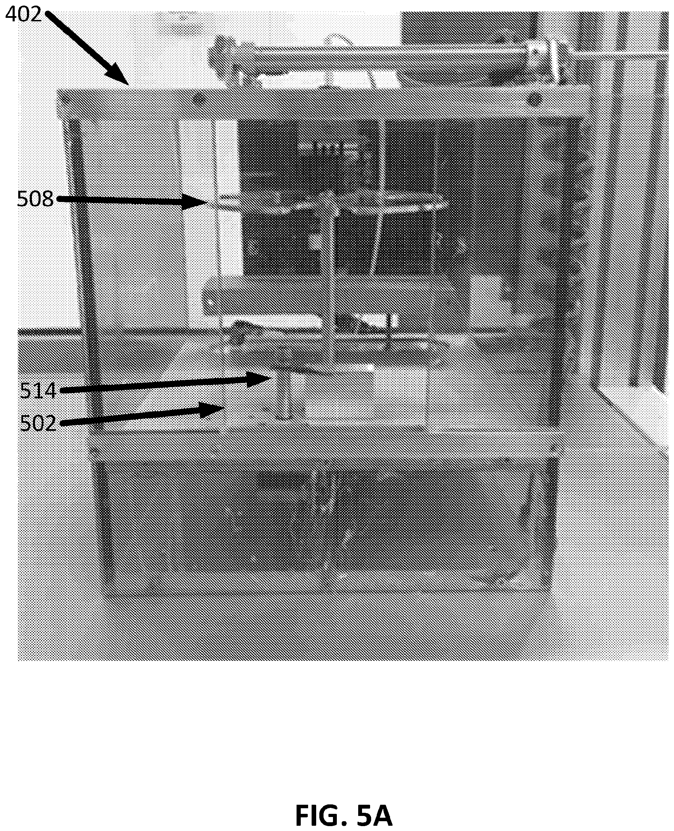

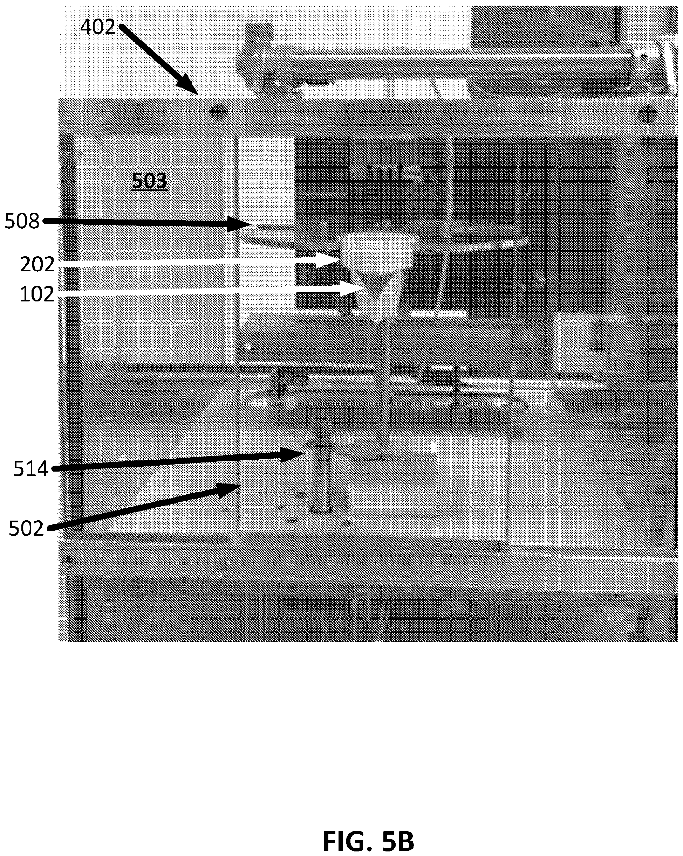

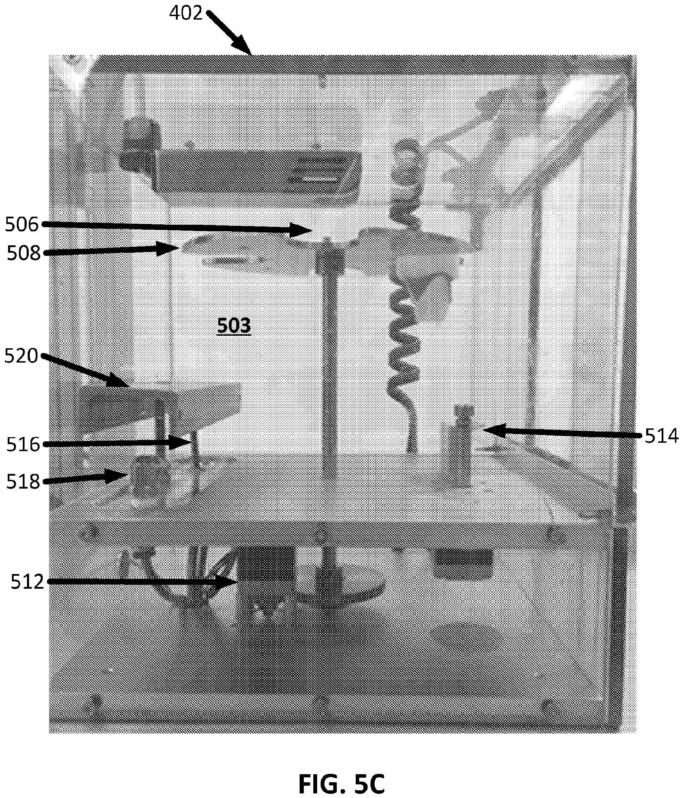

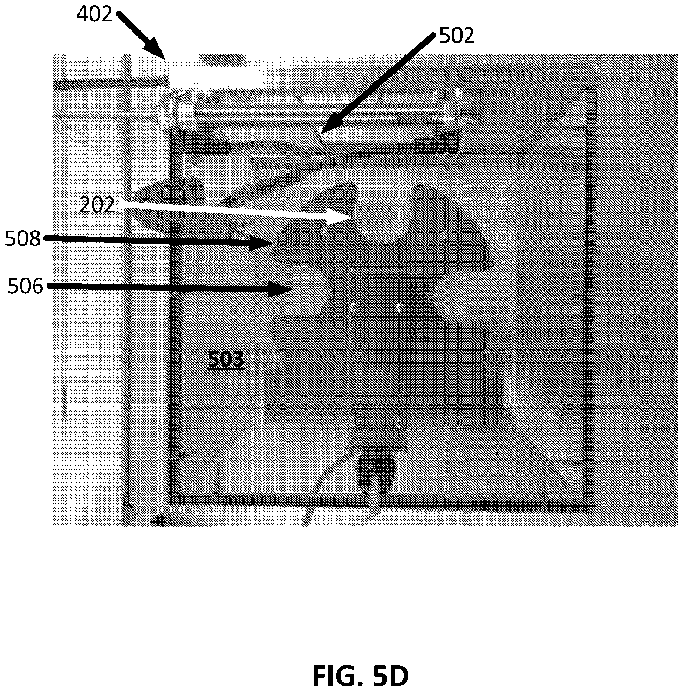

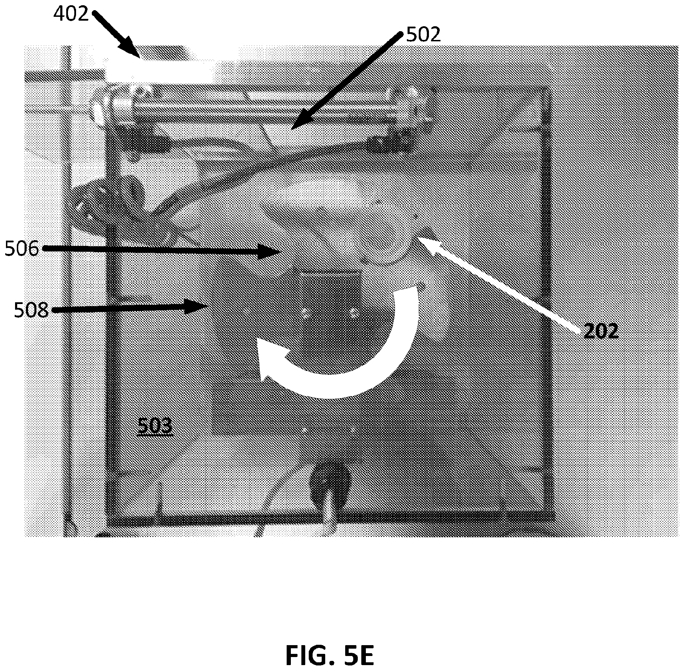

Referring back to first phase of curing 308 of methods 300 and 320, second chamber 402 can be configured as an environmental humidity chamber (EHC) 402. FIGs. 5A-5E are photographs of an example embodiment of an EHC 402. FIG. 5A is a front view of EHC 402, FIG. 5B is a front view of EHC 402 with an example embodiment of mandrel 202 having a valve 100 thereon, FIG. 5C is a side view of EHC 402 with mandrel 202 and valve 100, and FIGs. 5D and 5E are top down views of EHC 402 during rotation of mandrel 202 with valve 100. In some embodiments the leaflet polymer is transparent or translucent and thus, if frame 102 has been dipped, the presence of leaflets 110 may not be readily visible.

EHC 402 is a closeable chamber with one or more automatic doors 502 controllable for insertion and removal of valves 100. Immediately after dipping step 306, valve 100 (frame 102 with wet polymer in the form of nascent leaflets 110) and mandrel 202 can be inserted through door 502 into a holder, receptacle, or station 506 within the interior space 503 (the processing space between the walls, floor and ceiling) of EHC 402, as shown in FIG. 5B. In some embodiments, door 502 is a piston activated, or pneumatic, slide door. Door 502 can transition from closed to open and vice versa relatively quickly, for example, in about 15 seconds or less, which can ensure that the humidity level does not vary significantly when opened to the ambient atmosphere 410 of facility 400. In addition, the small volume of the EHC 402 allows for rapid recovery of the relative humidity level to a desired set level.

EHC 402 can include one or more environmental sensors 504 (FIG. 5C) for measuring temperature, humidity, pressure, or other factors. EHC 402 can be capable of controlling or maintaining the level of humidity within the internal space 503 with high precision. For example, in certain embodiments, the humidity in EHC 402 may be controlled to remain in a range of about ± 1% while valve 100 is in EHC 402 in closed state. The length of exposure of valve 100 within space 503 can be timed with high precision. For example, the exposure time can be controlled by processing circuitry 422.

The control of both the humidity level and exposure time can be critical to the manufacture of leaflets 110 with the desired thickness. It has been shown that leaflets 110 on the same valve 100 processed within EHC 402 can, when measured after final curing (e.g., after removal from second chamber 403 during a subsequent inspection) have leaflet thickness that are within 10 microns (or less) (e.g., 0-10 microns) of each other (i.e., leaflet-to-leaflet thickness variation on a single valve is less than 10 microns). This is for leaflets that are generally fabricated to have a final thickness in the range between 70 and 200 microns. In some embodiments it has been found that close control of these factors can result in repeatable valve leaflets with mean thicknesses within 2-5 microns of each other. In contrast, prosthetic heart valves made with tissue leaflets often have a leaflet-to-leaflet variation of 100 microns.

In some embodiments, the relative humidity (RH) within EHC 402 can be in the range of between about 20% to about 95%, or about 30% to about 90%, and more preferably, between about 50% to about 85%. In some embodiments, the exposure time can be between about 2 minutes to 20 minutes, or about 3 minutes to 15 minutes, and more preferably, between about 5 minutes to 10 minutes.

EHC 402 can include a movable fixture 508 having one or more (in this example four) holders 506, each configured to grasp and/or hold a mandrel 202 while valve 100 is situated thereon with wet polymer. In other embodiments, holders 506 can hold valve 100 directly. A motor 512 can move fixture 508 while valves 100 are within holders 506. In FIG. 5D, two of the four holders 506 are empty, one holder 506 is occupied by mandrel 202, and the other holder 506 is hidden from view. In this embodiment, fixture 508 includes a plate or shelf coupled to an axle, and the axle is coupled to motor 512, which is configured to rotate the axle and plate while mandrels 202 are placed thereon so that, e.g., each valve 100 experiences the same average environmental exposure within EHC 402. Mandrels 202 in fixture 508 can be moved in a plane perpendicular to the axis of rotation around EHC 402 (FIG. 5E), during which moving the polymer deposited on frame 102 and mandrel 202 partially cures. As they rotate around a path, for example, of about 15.24cm (6 inches) in diameter, they mark-out a cylindrical space occupying approximately 57% of the cross-sectional area of EHC 402. Viewed in this fashion, in some embodiments, at least about 50% of the cross section can be covered. In some embodiments, one to four stations 506 can concurrently process valves 100, but in other embodiments more than four stations 506 can be used concurrently.

During rotation, liquid polymer can runoff from mandrels 202, and can result in long strands of semi-solidified polymer that extend from the base of mandrel 202, sometimes all the way to the floor of EHC 402. EHC 402 can be equipped with an automated cutting or knife system 514 (FIG. 5B) that can be used to trim this excessive drip, either by cutting in the location shown in FIG. 5B, by lowering valve 100 to this location, or by raising cutting system 514 to the terminus of tip portion 208 of mandrel and cutting there. This minimizes further potential dripping or spillage or distortion to leaflets 110 during the second curing process 310.

In some embodiments, the duration of rotation can range from about 1 to 30 minutes per cycle, such as 5, 7.5, or 10 minutes per cycle. During this time each valve 100 can be rotated once or more about the interior of EHC 402. In certain embodiments, each valve 100 can be rotated about 720 degrees (two rotations) in approximately 10 minutes or less. This was found to result in highly consistent inter leaflet and intra valve leaflet thickness. For example, in certain embodiments, the standard deviation across three leaflets 110 was in the range of about 0-5 microns, whereas without rotation, one of the leaflets was found to be consistently systematically thicker that the other two by as much as 30 microns on average.

Interior space 503 of EHC 402 can have a relatively small volume. For example, inner space 503 of EHC 402 can be approximately 22.86 x 22.86 x 17.78cm (9 x 9 x 7 inches). By way of reference, some conventional approaches utilize a manufacturing chamber of approximately 121.92 x 121.92 x 91.44cm (48 x 36 x 36 inches) in which multiple steps of valve manufacturing are concurrently performed in the same ambient atmosphere. Inner space 503 of EHC 402 can have a volume, in some embodiments, of 16,387.1 cubic cm (1000 cubic inches) or less. This smaller volume and purpose-dedicated EHC 402 can allow for more precise control of the relative humidity (RH) level and greater homogeneity of the layer of polymer used to form leaflets 110.

In some embodiments, humidity in EHC 402 can be controlled by a variable mixture of relatively dry and wet mediums such as, for example, nitrogen and distilled water. EHC 402 can include a controllable output 516 for the dry medium and a controllable output 518 for the relatively wet medium. In certain embodiments, the dry medium (e.g., nitrogen) can be supplied to EHC 402 at a level of between about 1 to 15 liters/min, or preferably at about 8 liters/min. The supply can be pressurized and, while a fan can be used in some cases, EHC 402 can be configured such that no gas is fan-driven within EHC 402 during the curing. The supply of dry medium can be bifurcated and fed to both outputs 516 and 518 (FIG. 5C). The dry medium can have an RH from 0-5% and be fed into interior space 503 to lower humidity. The wet medium (e.g., a combination of the dry medium and water) can be fed to interior space 503 and used to raise humidity A shield or cover 520 can be placed over outputs 516 and 518 to prevent the dry or wet mediums from coming into direct contact with the curing polymer. Such an approach can ensure the optimal operation and rapid recovery of the humidity (RH) to the desired set point. Control equipment 405 can be communicatively coupled with sensor 504 and can control outputs 516 and 518 to maintain humidity within about ± 1% of a target level. Control equipment 405 can also control and maintain the temperature within EHC 402 at a target level.

For manufacturing consistent nominal valves 100, some of the optimum parameters for certain embodiments were determined to be as follows: 80% RH and a 5 minute exposure time for 17mm and 19mm aortic valves, 80% RH and a 7.5 minute exposure time for 21mm aortic valves, 80% RH and a 10 minute exposure time for 23mm aortic valves, 80% RH and a 7.5 minute exposure time for 25mm aortic valves, and 80% RH and a 10 minute exposure time for 27mm aortic valves. Of course, the embodiments of systems, devices, and methods described herein are not limited to the described processing parameters as such can and will vary depending on the specifics of the implementation.

Computer system 420 and/or control equipment 405 can cause door 502 to open and close. System 420 and/or equipment 405 can also control the triggering or initiation of the rotation sequence, the speed of rotation and duration of exposure within EHC 402, the control of the environment within EHC 402 (e.g., operation of humidity adjustment lines 516 and 518), process readings from humidity sensor 504, and the operation of cutting system 514. When the exposure period is over, system 420 and/or equipment 405 can cause door 502 to be opened and each valve 100 to be removed by automated transfer equipment 430, and then transferred to the next stage, e.g., chamber 403, for second phase curing 310. Automated transfer equipment 430 can then transfer valves 100 out of facility 400 so that finishing and other processes can be performed on each valve 100 and the manufacturing process can be completed.

### Example Embodiments of Prosthetic Valve Manufacturing with an Applied Identifier

In the manufacturing embodiments described herein, as well as other manufacturing methods, an identifier can be applied, placed, or marked on a surface of frame 102 or valve 100. This identifier can represent information about the frame 102 or valve 100. The identifier is machine-readable, and can convey information via optical, infrared, or ultraviolet codes, or by radio frequency (RF) energy (e.g., an RFID or Near Field Communication (NFC) tag) or others.

In many embodiments, the identifier can be applied to the surface of frame 102 prior to application of the leaflet coating (e.g., through dip-coating step 306) and application of the leaflet undercoating (e.g., coating step 304) if one is applied. The one or more coatings can be relatively thin and/or can be translucent or transparent to optical light (or other frequencies of energy). The identifier can be visually, optically, and/or otherwise read through the overlying coating(s) and used to trace (e.g., mark the time and location of) frame 102 through each step of the manufacturing process that occurs while the identifier is in place. The identifier can also be read before, during, and/or after each manufacturing step in order to for the manufacturing equipment (e.g., automated transfer equipment 430, chambers 401-403) to load and execute software instructions particular to the manufacturing of each specific valve 100.

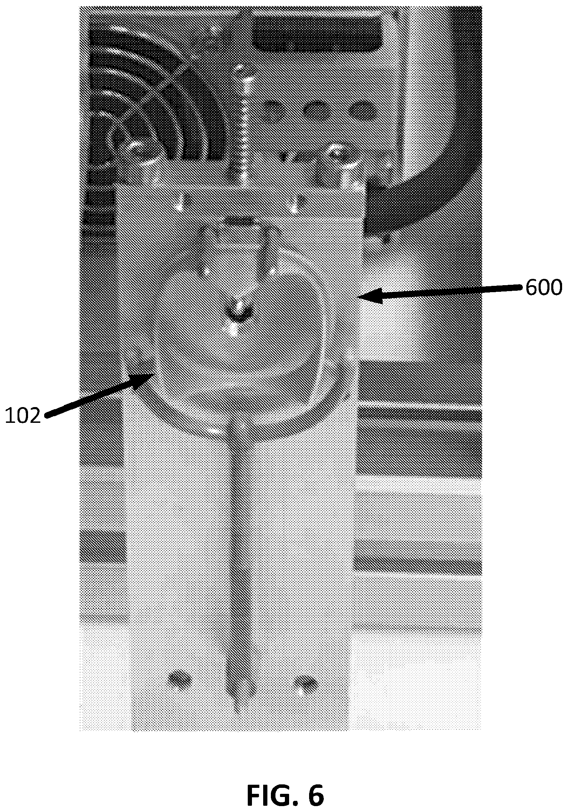

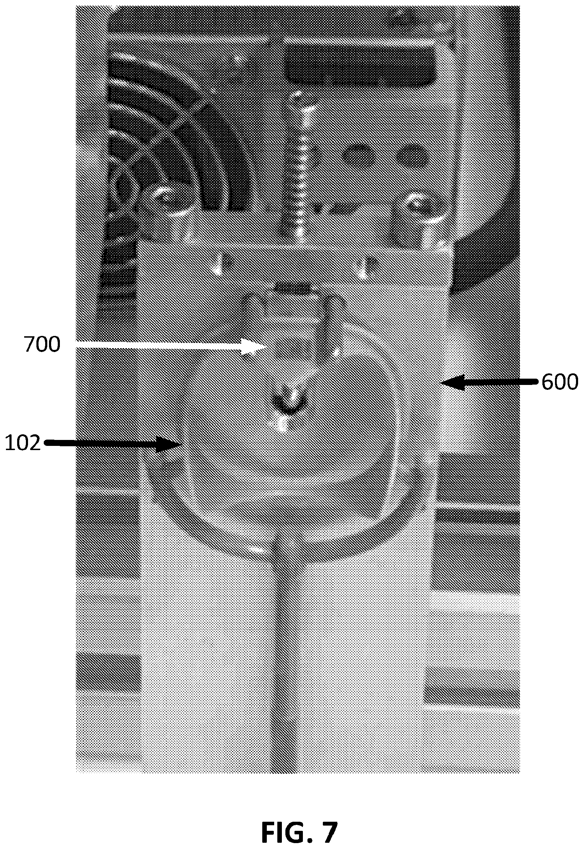

FIG. 6 is a photograph depicting an example embodiment of an unmarked frame 102 (e.g., a blank frame) in a fixture 600 (e.g., etching jig) used to hold frame 102 during application of an identifier marking directly to frame 102. FIG. 7 is a photograph depicting an example embodiment of a frame 102 after being marked with an optically scannable identifier 700. Various types of marking devices can be used that mark frame 102 with identifier 700 directly in or on the surface of frame 102. In some embodiments, the marking may be done by etching the surface, by applying heat so as to change the surface coloring, by applying a die or ink or other substance that is visually distinguishable from the surface of the frame 102, or any other suitable means of marking or a combination thereof.

In some embodiments, a carrier having identifier 700 thereon can be applied to the frame 102, such as a printed flexible patch or plate. In some embodiments, the carrier can be fastened to valve 100 using adhesive, bonding, a mechanical fixing member, or any combination thereof. Identifiers 700 that are another a substrate that can be fastened to frame 102 are particularly suitable when identifier 700 is in the form of an RFID or NFC tag.

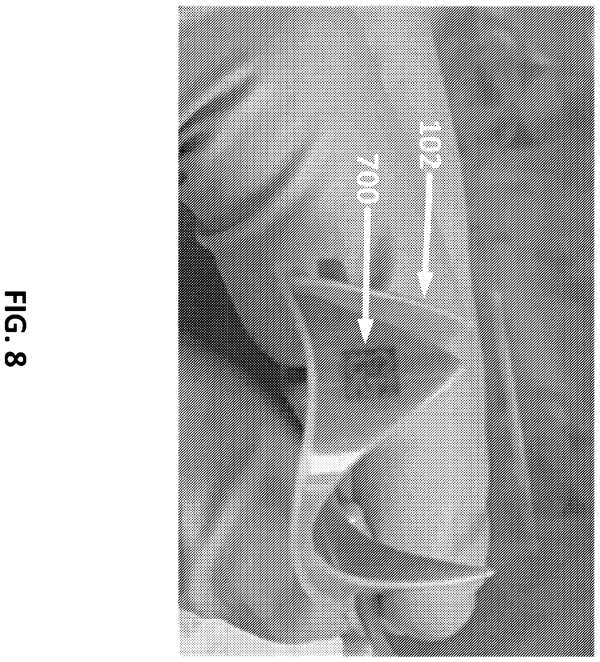

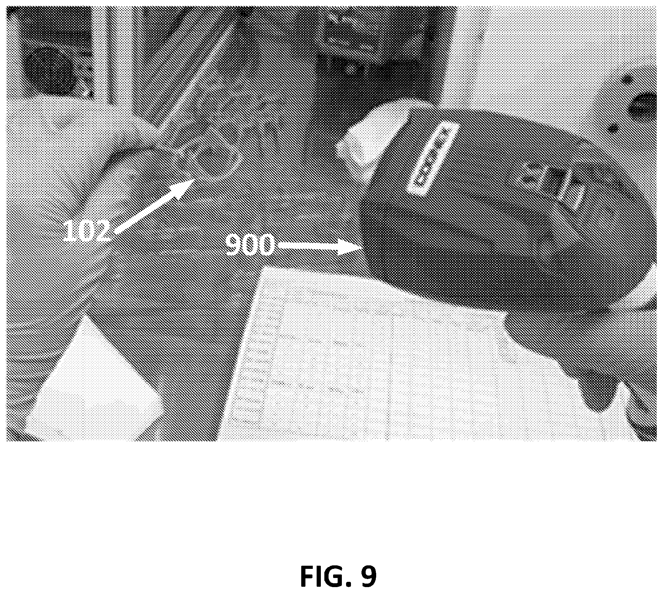

In the embodiment of FIG. 7, identifier 700 is in the form of a QR code and can be seen by virtue of its darker shading than the surface of frame 102 itself. FIG. 8 is a photograph depicting frame 102 with identifier 700 in more detail. FIG. 9 is a photograph depicting a process of reading identifier 700 using reader device 900, which in this embodiment is a scanner or camera for reading optical codes and/or texts. For example, the QR code can be read by a Cognex barcode scanning camera. In some embodiments, when the QR code is scanned, the read information is conveyed from scanner 900 to computer system 420 communicably connected thereto by a communicative coupling (which may be wired, or more preferably, wireless). The information read by reader device 900 can be processed or decoded by computer system 420.

Identifier 700 preferably identifies the individual valve 100 with enough specificity to distinguish that individual valve 100 from all other valves being processed by a particular piece of manufacturing equipment during a particular timeframe. Such an identifier 700 can be referred to herein as a "valve-specific identifier." For example, in certain embodiments, identifier 700 can be a unique identifier that has not been used with any other valve that has been processed by a piece of manufacturing equipment. In some embodiments, the identifier 700 can be recycled, so long as it is not reused within a time period in which the identity of two different valves 100 may be confused. In some embodiments, identifier 700 can be or include a unique serial number for the valve 100.

In some embodiments, identifier 700 can also include other information, such as "type" information that can include the identity of the native valve for which the prosthesis valve 100 serves as a replacement (e.g., aortic, mitral, etc.). In some embodiments, identifier 700 can include the size of valve 100 (e.g., 17 mm, 19 mm, 21 mm, 23 mm, 25 mm, 27 mm, etc.). In certain embodiments, identifier 700 includes a valve-specific identifier, type information (e.g., aortic, mitral, etc.), and size information (e.g., 17 mm, 19 mm, 21 mm, 23 mm, 25 mm, 27 mm, etc.). In certain embodiments, identifier 700 can include other information such as the type of material(s) being used to form the valve, or the type of surgical procedure in which the valve 100 may be used/recommended. In some embodiments, identifier 700 can omit a unique identifier and include only this "type" information.

In some embodiments, identifier 700 will at least uniquely identify valve 100 in all significant manufacturing stages that occur after association of identifier 700 with valve 100. Furthermore, in some embodiments, identifier 700 can be read after manufacturing is complete, i.e., post-manufacturing, such as after valve 100 has been distributed from the manufacturer to a distributor or other party responsible for selling valve 100 to the medical professional. Thus, any other third-party that comes into possession of valve 100 (e.g., distributor, retailer, shipper, medical professional, hospital, government agency, etc.) can read and utilize identifier 700 for tracking, inventory, analysis of clinical test results, or any other suitable purposes. The identity of valve 100 can be traced to the surgical procedure if read by medical professional or hospital having that capability. Thus, the identity of valve 100 can be associated with an identifier for a particular patient, such that performance of valve 100 in situ can be traced all of the way back to manufacturing stages. The identity of valve 100 can be ascertained after its removal from a patient, such as by any party capable of reading identifier 700, or by shipment of valve 100 back to the manufacturer for assessment.

Identifier 700 can be in an optically readable form. In some embodiments, the optical identifier 700 can take a host of different configurations. Identifier 700 can be a one-dimensional (1D) code (e.g., a barcode or dotcode, etc.), a two-dimensional (2D) code (e.g., a data matrix code, a Quick Response (QR) code, an Aztec code, etc.), a three-dimensional (3D) code (e.g., a colored 2D code, etc.), or any combination thereof. Identifier 700 can alternatively be a sequence or array of recognizable characters, such as alphanumeric characters and/or symbols. The information coded in identifier 700 can be recognized by scanning equipment 900, which may be in the form of an optical character recognition (OCR) or equivalent software or hardware (see, e.g., FIG. 9). Identifier 700 can also include two or more of the aforementioned media in combination (e.g., a 2D code with OCR text).

The embodiments of identifier 700 described herein are not limited to optically readable identifiers, and other types of identifiers can be used. For example, in certain embodiments, identifier 700 may be one that is readable using different frequencies of the electromagnetic spectrum, such as ultraviolet, infrared, radio frequency (e.g., RFID or near field communication (NFC) devices or tags), and so forth. These other forms or frequencies of identifiers can be used alone or in combination with optically readable forms described above.

While not limited to such, example embodiments will now be described where identifier 700 is placed on the surface of frame 102 prior to leaflet formation. In this embodiment, identifier 700 contains information that defines valve 100's type (such as aortic or mitral), size (e.g., 17mm to 27mm in increments of 2mm for Aortic, and 23 to 31mm in increments of 2mm for Mitral), and a valve-specific identifier (e.g., a unique serial number) that can be tracked throughout the entire manufacturing process.

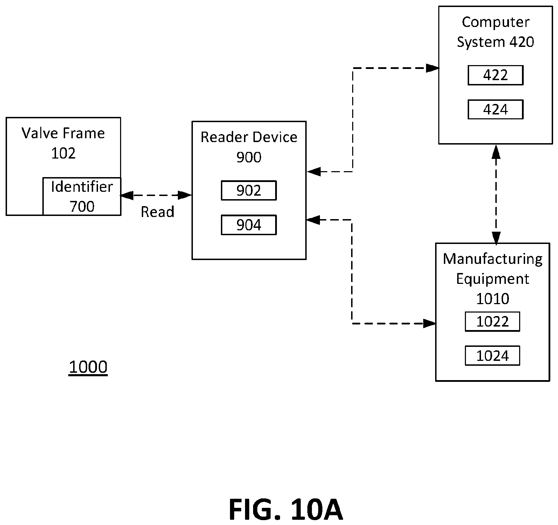

FIG. 10A is a block diagram depicting an example manufacturing environment 1000, including reader device 900, computer system 420, and manufacturing equipment 1010, each of which is communicatively coupled to the others. Reader device 900 is configured to read identifier 700 from valve frame 102 (in a state with or without leaflets). Reader device 900 includes processing circuitry 902 and non-transitory memory 904 that stores software instructions that, when executed by processing circuitry 902, cause reader device 900 to read identifier 700. The software instructions can also cause processing circuitry 902 to transfer the read information from identifier 702 to another system, such as computer system 420 and/or manufacturing equipment 1010, where the information can be further utilized in manufacturing.

Non-transitory memory 424 of computer system 420 includes software instructions that, when executed by processing circuitry 422, cause processing circuitry 422 to read the information communicated from reader device 900. The instructions can further cause processing circuitry 422 to transfer relevant portions of the information to manufacturing equipment 1010 for further use in manufacturing. The instructions can also or alternatively cause processing circuitry 422 to select an appropriate software program, function, or parameter for use in manufacturing valve 100 and either execute that program, function, or parameter, or transfer it to manufacturing equipment 1010 for execution.

Manufacturing equipment 1010 can include processing circuitry 1012 and non-transitory memory 1014. Non-transitory memory 1014 includes software instructions that, when executed by processing circuitry 1012, cause processing circuitry 1012 to read the information communicated from either reader device 900 or computer system 420. The instructions can further cause processing circuitry 1012 to select an appropriate software program, function, or parameter for use in manufacturing valve 100 and execute that program, function, or parameter. Manufacturing equipment 1010 can be, for example, control equipment 404, 405, 406, and/or 408 and the software program, function, or parameter can be for control of chambers 401, 402, 403, and/or facility 400. Manufacturing equipment 1010 can be, for example, automated transfer equipment 430 and the software program, function, or parameter can be for control of equipment 430 in moving valve 100. Manufacturing equipment 1010 can be, for example, cutting system 514 and the software program, function, or parameter can be for control of cutting system 514 in EHC 402. Manufacturing equipment 1010 can be, for example, equipment used in finishing valve 100 (e.g., trimming, sewing a cuff, measuring, inspecting, packaging) and the software program, function, or parameter can be for control of the finishing equipment.

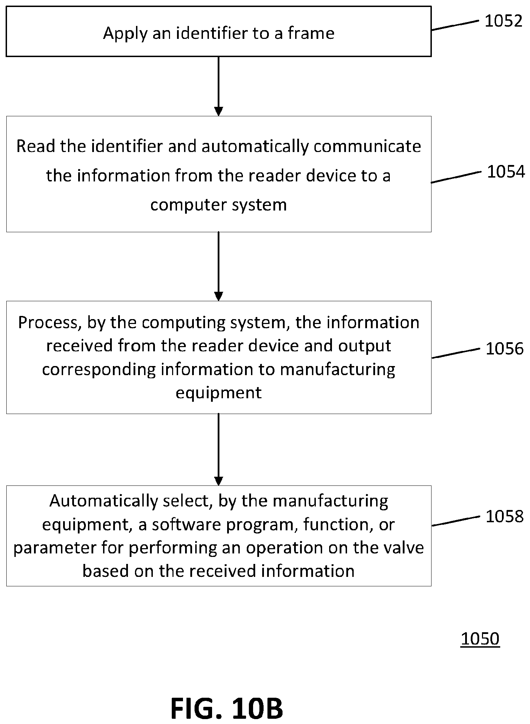

FIG. 10B is a flow diagram depicting an example embodiment of a method 1050 of utilizing identifier 700 during one or more manufacturing processes. At 1052, an identifier 700 is applied to a frame 102. At 1054, identifier 700 can be read or scanned with a reader device 900, and the read information can be automatically communicated by reader device 900 to computer system 420. In some cases, frame 102 can be coated, and reading identifier 700 can occur through the coating. At 1056, computer system 420 can process the information received from reader device 900 and output the information to manufacturing equipment 1010 (e.g., facility 400, chambers 401-403, automated equipment 430, etc.). Then, at 1058, manufacturing equipment 1010 can select a software program or function (or other parameters) for performing an operation or action on valve 100 based on the received information. In other embodiments, computer system 420 can select the appropriate software program, function, or parameters and output them directly to manufacturing equipment 1010. For example, computer system 420 can instruct particular equipment 1010 to execute a particular program.

The operation or action selected at 1058 to be performed on valve 100 can be a step in manufacturing valve 100 (e.g., coating frame 102 at step 304, dipping step 306, first phase curing step 308, second phase curing step 310, or a finishing step 312 (e.g., trimming, attaching a sewing cuff, visual inspection, packaging). For example, equipment associated with the valve dipping 306 can receive, from computer system 420, the valve 100's type and size to select a processing software program that ensures that the valve is dipped to the correct depth, at the correct speed, and remains dipped for the proper time, while exposed to the proper environmental conditions. Equipment associated with EHC 402 can receive, from computer system 420, the valve 100's type and size to select a processing software program that ensures that the valve is rotated at the correct speed, for the proper time, while exposed to the proper environmental conditions. Equipment associated with chamber 403 can receive, from computer system 420, the valve 100's type and size to select a processing software program that ensures that the valve is cured under the proper environmental conditions for the proper time. Equipment associated with valve trimming can receive, from computer system 420, the valve 100's size and type to ensure the correct program is executed to cut leaflets 110 (and mandrel 202) at the correct position. Equipment associated with a cuff sewing system can receive, from computer system 420, the valve 100's size and type to ensure the correct program is executed to suture the sewing cuff onto the finished valve prosthesis 100.

The operation or action can also be a step of moving valve 100 from one location to another, e.g., moving valve 100 from EHC 402 to chamber 403 with automated transfer equipment 430. This information can inform transfer equipment 430 of the precise location of valve 100 (as valves vary in size). The operation or action can also be a step of measuring valve 100 to ascertain a leaflet measurement (e.g., thickness).

For example, in some embodiments, prior to or upon entering a particular stage of manufacturing, valve 100 can be scanned either automatically or by a person using an automatic scanner 900 in step 1054. This information can be relayed to the computer system 420 in step 1056, which can then output that information or a portion thereof to the automated manufacturing equipment of the particular stage of manufacturing. The information passed to the manufacturing equipment can include additional information that is not coded by the identifier 700, such as a log file with in-line size measurements, or other measured or recorded variables that describe the condition of the valve 100 or the manner in which it has been processed thus far. The manufacturing equipment can utilize this information to set appropriate processing parameters for processing the valve 100 at a particular manufacturing stage.

Any measurement data collected by the manufacturing equipment can be relayed back to computer system 420, which can append that collected information to a log file for the particular valve 100. For example, in some embodiments, the identity of the manufacturing equipment, the time and date at which valve 100 was processed by the manufacturing equipment, a length of time in which valve 100 was processed by the manufacturing equipment, environmental conditions (e.g., temperature, pressure, humidity, and the like) recorded by the manufacturing equipment during processing of valve 100, and other variables can be output by the manufacturing equipment to computer system 420 in a manner that associates that information with the identity of the particular valve 100 (or valves 100 being processed, if concurrent batch processing occurs).

Upon exiting a given manufacturing stage, valve 100 can be scanned again so as to track valve 100's location and time of exit. Then, upon reaching the next stage, valve 100 can be scanned again. This process can repeat for each stage of manufacturing. This can occur one or more times depending on the number of stages of manufacturing used to fabricate valve 100, or the number of stages of manufacturing in which specific valve identity is desired to be utilized. Valve 100 does not need to be scanned at every stage, for example, in those stages where a linear processing line exists such that the manufacturing equipment knows with certainty the identify of each valve 100.

For example, in some embodiments, valve 100 can be scanned upon entering a measuring stage. A Digital Measuring System (DMS) can receive, from computer system 420, the valve 100's type, size and/or serial number to ensure that both the correct measurement program can be selected for that particular valve 100 by the DMS and that the data is either treated as a master measurement for that valve 100 (e.g., a measurement prior to dipping 306 and leaflet formation) or a thickness measurement (e.g., a measurement after curing 310) where the thickness of leaflets 110 can be ascertained.

Throughout the entire manufacturing process, the output data as well as the date and time of every operation is continuously recorded on a digital structured query language (SQL) server to create and update valve 100's log file (e.g., digital design history record).

The embodiments of valve 100 described herein are suitable for implantation in the body of a patient using any number of medical procedures. Preferably, these embodiments of valve 100 are for direct implantation into the body using open heart surgery. Such embodiments of valve 100 are not radially collapsible for insertion into an intravascular delivery device (e.g., a catheter) or a transapical delivery device. However, in other embodiments, valve 100 can be configured with a radially collapsible support structure 102 that allows the lateral dimension of valve 100 to be reduced by a degree sufficient to permit the insertion into an appropriately sized intravascular or transapical delivery device.

Example embodiments of systems, devices, and methods are described herein that relate to the manufacture of prosthetic heart valves. However, the scope of the present disclosure is not limited to such, and can likewise be applied to prosthetics for replacement of other valves in other locations in the patient's body outside of the heart.

In all the embodiments described herein, automated steps are performed by processing circuitry executing software instructions. The software instructions can be stored in non-transitory memory. The processing circuitry and non-transitory memory can be part of a single discrete system, such as a workstation or server, or can be distributed across multiple systems or devices, such as a computer system accessing remotely stored information across a network. The processing circuitry can be a microprocessor, array of processors, programmable logic controller (PLC), circuitry distributed across multiple different devices, or others. The non-transitory memory can be volatile or nonvolatile memory, can be integrated on the same chip as the processing circuitry, a different chip, distributed across multiple chips, or others.

The embodiments described herein are restated and expanded upon in the following paragraphs without explicit reference to the figures. In many example embodiments, a method of valve leaflet manufacture is provided, including: applying polymer in a liquid state to a valve frame and a leaflet formation structure; loading the valve frame and the leaflet formation structure, with the polymer in the liquid state, into a chamber; and automatically moving the valve frame and the leaflet formation structure around the chamber for a period of time during which the polymer at least partially cures.

In some embodiments, applying polymer in a liquid state to the valve frame and the leaflet formation structure includes: dip-coating the valve frame by submerging at least a portion of the valve frame and the leaflet formation structure in a reservoir filled with the polymer in the liquid state; and removing the at least the portion of the valve frame and the leaflet formation structure from the reservoir.

In some embodiments, automatically moving the valve frame and the leaflet formation structure around the chamber for the period of time during which the polymer at least partially cures includes: automatically rotating the valve frame and the leaflet formation structure around the chamber in a plane perpendicular to an axis. The valve frame and the leaflet formation structure can be rotated at least one full rotation about the axis.

In some embodiments, the chamber is an environmental humidity chamber (EHC).

In some embodiments, the method further includes: opening a door to the chamber prior to loading the valve frame and the leaflet formation structure; and closing the door to the chamber after loading the valve frame and the leaflet formation structure.

In some embodiments, automatically moving the valve frame and the leaflet formation structure around the chamber includes moving the valve frame and the leaflet formation structure through at least 50% of a cross sectional area of the chamber.

In some embodiments, no gas is fan-driven within the chamber during the period of time.

In some embodiments, the method further includes removing the valve frame and the leaflet formation structure from the chamber.

In some embodiments, the method further includes cutting excess at least partially cured polymer from the valve frame.

In some embodiments, when the chamber is closed, and the valve frame is automatically moved around the chamber, the humidity in the chamber is controlled to ± 1%.

In some embodiments, the chamber has a volume of 16,387.1 cubic cm (1000 cubic inches) or less.

In some embodiments, the valve frame has an optical identifier thereon.

In some embodiments, the chamber is a first chamber, and the method further includes: removing the valve frame and the leaflet formation structure from the first chamber after the period of time, where the polymer is only partially cured; inserting the valve frame and the leaflet formation structure into a second chamber; and heating the valve frame and the leaflet formation structure in the second chamber until the polymer cures. The method can further include trimming cured polymer to form a plurality of leaflets on the valve frame. After removal from the second chamber the polymer that will form a plurality of leaflets on the valve structure is between 70 and 200 microns in thickness.

In some embodiments, the valve frame is a first valve frame and the leaflet formation structure is a first leaflet formation structure, and the method further includes: applying polymer in the liquid state to a second valve frame and a second leaflet formation structure; loading the second valve frame and the second leaflet formation structure, with the polymer in the liquid state, into the chamber; and automatically moving the first valve frame, the first leaflet formation structure, the second valve frame, and the second leaflet formation structure around the chamber for the period of time during which the polymer on the first valve frame, the first leaflet formation structure, the second valve frame, and the second leaflet formation structure at least partially cures. The method can further include: removing the second valve frame and the second leaflet formation structure from the first chamber after the period of time, where the polymer is only partially cured; inserting the second valve frame and the second leaflet formation structure into the second chamber; heating the second valve frame and the second leaflet formation structure in the second chamber until the polymer cures; and trimming cured polymer to form a plurality of leaflets on the second valve frame. A variation in thickness between each of the plurality of leaflets on the first valve frame and each of the plurality of leaflets on the second valve frame can be less than 10 microns in thickness.

In many embodiments, a method of valve leaflet manufacture is provided, the method including: applying polymer in a liquid state to a valve frame and a leaflet formation structure; loading the valve frame and the leaflet formation structure, with the polymer in the liquid state, into a first chamber and partially curing the polymer while the valve frame and the leaflet formation structure are within the first chamber; and loading the valve frame and the leaflet formation structure, with the polymer in a partially cured state, into a second chamber and curing the polymer while the valve frame and the leaflet formation structure are within the second chamber.

In some embodiments, applying polymer in a liquid state to the valve frame and the leaflet formation structure includes: dip-coating the valve frame by submerging at least a portion of the valve frame and the leaflet formation structure in a reservoir filled with the polymer in the liquid state; and removing the at least the portion of the valve frame and the leaflet formation structure from the reservoir.

In some embodiments, the method further includes automatically moving the valve frame and the leaflet formation structure around the first chamber for a period of time during which the polymer partially cures. Automatically moving the valve frame and the leaflet formation structure around the first chamber can further include moving the valve frame and the leaflet formation structure through at least 50% of a cross sectional area of the chamber.

In some embodiments, the method further includes automatically rotating the valve frame and the leaflet formation structure around the first chamber in a plane perpendicular to an axis for a period of time during which the polymer partially cures. The valve frame and the leaflet formation structure can be rotated at least one full rotation about the axis. Automatically rotating the valve frame and the leaflet formation structure around the first chamber can include moving the valve frame and the leaflet formation structure through at least 50% of a cross sectional area of the chamber.

In some embodiments, the first chamber is an environmental humidity chamber (EHC).

In some embodiments, no gas is fan-driven within the first chamber for a period of time during which the polymer partially cures.

In some embodiments, the method further includes removing the valve frame and the leaflet formation structure from the chamber.

In some embodiments, the method further includes cutting excess partially cured polymer from the valve frame prior to loading the valve frame and the leaflet formation structure into the second chamber.

In some embodiments, when the first chamber and the polymer is partially curing, the humidity in the chamber is controlled to ± 1%.

In some embodiments, the method further includes the first chamber has a volume of 16,387.1 cubic cm (1000 cubic inches) or less.

In some embodiments, the valve frame has an optical identifier thereon.

In some embodiments, the method further includes trimming cured polymer to form a plurality of leaflets on the valve frame.

In some embodiments, the valve frame is a first valve frame and the leaflet formation structure is a first leaflet formation structure, and the method further includes: applying polymer in a liquid state to a second valve frame and a second leaflet formation structure; loading the second valve frame and the second leaflet formation structure, with the polymer in the liquid state, into the first chamber and partially curing the polymer while the first valve frame, the first leaflet formation structure, the second valve frame, and the second leaflet formation structure are within the first chamber; and loading the second valve frame and the second leaflet formation structure, with the polymer in a partially cured state, into a second chamber and curing the polymer while the first valve frame, the first leaflet formation structure, the second valve frame, and the second leaflet formation structure are within the second chamber. The method can further include trimming cured polymer to form a plurality of leaflets on the second valve frame. The variation in thickness between each of the plurality of leaflets on the first valve frame and each of the plurality of leaflets on the second valve frame can be less than 10 microns in thickness. The method can include removing the valve frame and leaflet formation structure from the second chamber. After removal from the second chamber the polymer on the leaflet formation structure can be between 70 and 200 microns in thickness.

In many embodiments, an apparatus for partial curing of polymer is provided, the apparatus including: a chamber; a movable fixture in the chamber, the movable fixture including at least one receptacle; a motor coupled with the movable fixture and configured to cause the fixture to move; a humidity sensor in the chamber; a first controllable output in the chamber, the first controllable output configured to controllably dispense a first medium into the chamber; a second controllable output in the chamber, the second controllable output configured to dispense a second medium in the chamber, the first medium having a higher relative humidity than the second medium; and a control system communicatively coupled with the humidity sensor and the first and second controllable outputs, the control system programmed to control humidity within the chamber.

In some embodiments, the receptacle is configured to hold a valve frame or a leaflet formation structure.

In some embodiments, the movable fixture includes a plurality of receptacles, each being configured to hold a valve frame or a leaflet formation structure.

In some embodiments, the movable fixture is configured to rotate each receptacle through a plane perpendicular to an axis.

In some embodiments, the movable fixture is configured to rotate, and the motor is a rotation motor.

In some embodiments, the movable fixture is adapted to move the receptacle through at least 50% of a cross sectional area of the chamber.

In some embodiments, the apparatus further includes a cutting system. The cutting system can be configured to cut partially cured polymer.

In some embodiments, the control system is programmed to control humidity within the chamber to within ± 1% of a humidity setting.

In some embodiments, the chamber has a volume of 16,387.1 cubic cm (1000 cubic inches) or less.

In some embodiments, the chamber includes a door. The door can be pneumatically actuatable.

In many embodiments, a method of identifying a prosthetic valve is provided, the method including: marking a valve frame of the prosthetic valve with an identifier; coating the valve frame and identifier with a coating; and reading the identifier through the coating, where the identifier is read by a machine.

In some embodiments, reading the identifier includes reading information coded in the identifier by scanning equipment; and automatically communicating the information from the scanning equipment to a computer system over a communicative coupling. The scanning equipment can include processing circuitry and non-transitory memory on which a plurality of instructions are stored that, when executed by the processing circuitry, cause the processing circuitry to cause communication of the information from the scanning equipment to the computer system. The method can further include: processing, by the computer system, the information received from the scanning equipment and outputting corresponding information to manufacturing equipment. The method can further include: automatically selecting, by the manufacturing equipment, a software program, function, or parameter for performing an operation or act on the prosthetic valve based on the corresponding information. The computer system can include processing circuitry and non-transitory memory on which a plurality of instructions are stored that, when executed by the processing circuitry, cause the processing circuitry to process the information received from the scanning equipment and output corresponding information to the manufacturing equipment. The manufacturing equipment can include processing circuitry and non-transitory memory on which a plurality of instructions are stored that, when executed by the processing circuitry, cause the processing circuitry to automatically select a software program for performing a manufacturing operation on the valve based on the corresponding information.

In some embodiments, the identifier is optically readable. The identifier can be at least one of the following: a one-dimensional code, a two-dimensional code, a three-dimensional code, a sequence of optically recognizable alphanumeric or symbolic characters.

In some embodiments, the identifier is a QR code.

In some embodiments, the identifier represents information about the valve prosthesis. The information can include at least one of: a valve type, a valve size, or a serial number. The information can include a valve type, a valve size, and a serial number. The valve type can be either aortic or mitral. The valve size can be one of: 17mm, 19mm, 21mm, 23mm, 25mm, 27mm, or 29mm.

In some embodiments, the method further includes: automatically selecting a software program, function, or parameter for an automated operation or act to be performed on the valve prosthesis based on information read from the identifier. The software program, function, or parameter can be for applying a polymer to the coated valve frame and a leaflet formation structure, where the polymer is for formation of a plurality of leaflets. The method can further include reading the identifier through the coating and the polymer. The software program, function, or parameter can be for an automated first phase of curing a polymer on the valve frame and on a leaflet formation structure. The software program, function, or parameter can be for an automated second phase of curing a polymer on the valve frame and on a leaflet formation structure. The software program, function, or parameter can be for trimming a cured polymer to form a plurality of leaflets on the valve frame. The software program, function, or parameter can be for measuring a plurality of leaflets on the valve frame. The software program, function, or parameter can be for automatically transferring the valve frame between manufacturing stages. The software program, function, or parameter can be for finishing the valve frame.

In many embodiments, a prosthetic valve is provided that includes: a frame having an identifier thereon, the identifier being machine readable; and a plurality of polymeric leaflets integrally coupled with the frame by a layer of polymer, where the layer of polymer covers the identifier and where the identifier remains machine readable through the layer of polymer.

In some embodiments, the identifier represents at least a unique identifier of the prosthetic valve.

In some embodiments, the identifier represents at least a serial number of the prosthetic valve.

In some embodiments, the identifier represents at least a unique identifier and a type of the prosthetic valve.

In some embodiments, the identifier represents at least a unique identifier and a size of the prosthetic valve.

In some embodiments, the identifier represents at least a unique identifier, a type of the prosthetic valve, and a size of the prosthetic valve.

In some embodiments, the identifier is an optically readable identifier. The identifier can be at least one of the following: a one-dimensional code, a two-dimensional code, a three-dimensional code, a sequence of optically recognizable alphanumeric or symbolic characters. The optical identifier can be a QR code.

In some embodiments, the frame and the identifier are covered by a coating and the layer of polymer over the coating, where the identifier remains machine readable through both the coating and the layer of polymer.

In some embodiments, the identifier is readable with radio frequency (RF) energy.

In some embodiments, the prosthetic valve is a prosthetic heart valve. The identifier can represent at least a type of the prosthetic heart valve. The type of the prosthetic heart valve can be either aortic or mitral.

Various aspects of the present subject matter are set forth below, in review of, and/or in supplementation to, the embodiments described thus far, with the emphasis here being on the interrelation and interchangeability of the following embodiments. In other words, an emphasis is on the fact that each feature of the embodiments can be combined with each and every other feature unless explicitly stated otherwise or logically implausible.

Those of ordinary skill in the art will readily recognize, in light of this description, the many variations of suitable dip casting procedures, pressures, and temperatures that are not stated here yet are suitable to fabricate the prosthetic heart valves described herein. Likewise, those of ordinary skill in the art will also recognize, in light of this description, the alternatives to dip casting that can be used to fabricate the prosthetic heart valves described herein.

As used herein and in the appended claims, the singular forms "a", "an", and "the" include plural referents unless the context clearly dictates otherwise.

Where a range of values is provided, each intervening value, to the tenth of the unit of the lower limit unless the context clearly dictates otherwise, between the upper and lower limit of that range and any other stated or intervening value in that stated range, is encompassed within the disclosure and can be claimed as a sole value or as a smaller range. Where the stated range includes one or both of the limits, ranges excluding either or both of those included limits are also included in the disclosure.

Where a discrete value or range of values is provided, that value or range of values may be claimed more broadly than as a discrete number or range of numbers, unless indicated otherwise. For example, each value or range of values provided herein may be claimed as an approximation and this paragraph serves as antecedent basis and written support for the introduction of claims, at any time, that recite each such value or range of values as "approximately" that value, "approximately" that range of values, "about" that value, and/or "about" that range of values. Conversely, if a value or range of values is stated as an approximation or generalization, e.g., approximately X or about X, then that value or range of values can be claimed discretely without using such a broadening term.

However, in no way should this specification be interpreted as implying that the subject matter disclosed herein is limited to a particular value or range of values absent explicit recitation of that value or range of values in the claims. Values and ranges of values are provided herein merely as examples.

## Claims

1. A method of valve leaflet (110) manufacture, comprising:
applying polymer in a liquid state to a valve frame (102) and a leaflet formation structure (202) by dip-coating the valve frame (102) by submerging at least a portion of the valve frame (102) and the leaflet formation structure (202) in a reservoir filled with the polymer in the liquid state;
loading the valve frame (102) and the leaflet formation structure (202), with the polymer in the liquid state, into a first environmental humidity chamber (402) having a humidity sensor (504) and control equipment (405) to maintain a controlled humidity environment greater than 50% and less than 95%;
automatically moving the valve frame (102) and the leaflet formation structure (202) around the environmental humidity chamber (402) for a period of time during which the humidity within the environmental humidity chamber (402) is controlled and during which the polymer at least partially cures; the method further comprising:
removing the valve frame (102) and the leaflet formation structure (202) from the first environmental humidity chamber (402) after the period of time, wherein the polymer is only partially cured;
inserting the valve frame (102) and the leaflet formation structure (202) into a second environmental humidity chamber (403); and
heating the valve frame (102) and the leaflet formation structure (202) in the second environmental humidity chamber (403) until the polymer cures.

2. The method of claim 1, wherein automatically moving the valve frame (102) and the leaflet formation structure (202) around the environmental humidity chamber (402) for the period of time during which the polymer at least partially cures comprises:
automatically rotating the valve frame (102) and the leaflet formation structure (202) around the environmental humidity chamber (402) in a plane perpendicular to an axis.

3. The method of claim 2, wherein the valve frame (102) and the leaflet formation structure (202) are rotated at least one full rotation about the axis.

4. The method of claim 1, wherein the controlled humidity environment of the environmental humidity chamber (402) is operably connected to a wet medium as a source of humidity.

5. The method of claim 1, further comprising:
opening a door to the environmental humidity chamber (402) prior to loading the valve frame (102) and the leaflet formation (202) structure; and
closing the door to the environmental humidity chamber (402) after loading the valve frame (102) and the leaflet formation (202) structure.

6. The method of claim 1, wherein automatically moving the valve frame (102) and the leaflet formation structure (202) around the environmental humidity chamber comprises moving the valve frame (102) and the leaflet formation structure (202) through at least 50% of a cross sectional area of the environmental humidity chamber (402).

7. The method of claim 1, wherein no gas is fan-driven within the environmental humidity chamber (402) during the period of time.

8. The method of claim 1, further comprising removing the valve frame (102) and the leaflet formation structure (202) from the environmental humidity chamber (402).

9. The method of claim 1, further comprising cutting excess at least partially cured polymer from the valve frame (102).

10. The method of claim 1, wherein, when the environmental humidity chamber (402) is closed and the valve frame (102) is automatically moved around the environmental humidity chamber (402), and wherein the humidity in the environmental humidity chamber (402) is controlled to ± 1% within the controlled humidity environment greater than 50% and less than 95%.

11. The method of claim 1, wherein the environmental humidity chamber (402) has a volume of 16,387.1 cubic centimetres (1000 cubic inches) or less.

12. The method of claim 1, wherein the valve frame (102) has a machine-readable optical identifier thereon.

13. The method of claim 1, further comprising trimming cured polymer to form a plurality of leaflets (110) on the valve frame (102).

14. The method of claim 1, wherein after removal from the second chamber (403) the polymer that will form a plurality of leaflets (110) on the valve structure is between 70 and 200 microns in thickness.

15. The method of claim 1, wherein the valve frame (102) is a first valve frame (102) and the leaflet formation structure (202) is a first leaflet formation structure (202), the method further comprising:
applying polymer in the liquid state to a second valve frame (102) and a second leaflet formation structure (202);
loading the second valve frame (102) and the second leaflet formation structure (202), with the polymer in the liquid state, into the environmental humidity chamber (402); and
automatically moving the first valve frame (102), the first leaflet formation structure (202), the second valve frame (102), and the second leaflet formation structure (202) around the environmental humidity chamber (402) for the period of time during which the polymer on the first valve frame (102), the first leaflet formation structure (202), the second valve frame (102), and the second leaflet formation structure (202) at least partially cures; the method optionally further comprising:
removing the second valve frame (102) and the second leaflet formation structure (202) from the environmental humidity chamber (402) after the period of time, wherein the polymer is only partially cured;
inserting the second valve frame (102) and the second leaflet formation structure (202) into a second environmental humidity chamber (403);
heating the second valve frame (102) and the second leaflet formation structure (202) in the second environmental humidity chamber (403) until the polymer cures; and
trimming cured polymer to form a plurality of leaflets (110) on the second valve frame (102); optionally wherein
variation in thickness between each of the plurality of leaflets (110) on the first valve frame (102) and each of the plurality of leaflets (110) on the second valve frame is less than 10 microns in thickness.

## Patentansprüche

1. Verfahren zur Herstellung eines Klappensegels (110), umfassend:
Aufbringen eines Polymers in einem flüssigen Zustand auf einen Klappenrahmen (102) und eine Segelbildungsstruktur (202) durch Tauchbeschichten des Klappenrahmens (102) durch Eintauchen mindestens eines Teils des Klappenrahmens (102) und der Segelbildungsstruktur (202) in ein Reservoir, das mit dem Polymer im flüssigen Zustand gefüllt ist;
Laden des Klappenrahmens (102) und der Segelbildungsstruktur (202) mit dem Polymer im flüssigen Zustand in eine erste Umgebungsfeuchtekammer (402) mit einem Feuchtesensor (504) und einer Steuereinrichtung (405), um eine Umgebung mit kontrollierter Feuchte von mehr als 50 % und weniger als 95 % aufrechtzuerhalten;
automatisches Bewegen des Klappenrahmens (102) und der Segelbildungsstruktur (202) durch die Umgebungsfeuchtekammer (402) für einen Zeitraum, während dem die Feuchte innerhalb der Umgebungsfeuchtekammer (402) kontrolliert wird und während dem das Polymer zumindest zum Teil aushärtet; wobei das Verfahren weiterhin umfasst:
Herausnehmen des Klappenrahmens (102) und der Segelbildungsstruktur (202) aus der ersten Umgebungsfeuchtekammer (402) nach dem Zeitraum, wobei das Polymer nur zum Teil ausgehärtet ist;
Einbringen des Klappenrahmens (102) und der Segelbildungsstruktur (202) in eine zweite Umgebungsfeuchtekammer (403); und
Erhitzen des Klappenrahmens (102) und der Segelbildungsstruktur (202) in der zweiten Umgebungsfeuchtekammer (403), bis das Polymer aushärtet.

2. Verfahren nach Anspruch 1, wobei das automatische Bewegen des Klappenrahmens (102) und der Segelbildungsstruktur (202) durch die Umgebungsfeuchtekammer (402) für den Zeitraum, während dem das Polymer zumindest zum Teil aushärtet, umfasst:
automatisches Drehen des Klappenrahmens (102) und der Segelbildungsstruktur (202) durch die Umgebungsfeuchtekammer (402) in einer Ebene, die senkrecht zu einer Achse ist.

3. Verfahren nach Anspruch 2, wobei der Klappenrahmen (102) und die Segelbildungsstruktur (202) mindestens eine volle Umdrehung um die Achse gedreht werden.

4. Verfahren nach Anspruch 1, wobei die Umgebung mit kontrollierter Feuchte der Umgebungsfeuchtekammer (402) betriebsfähig mit einem nassen Medium als eine Feuchtequelle verbunden ist.

5. Verfahren nach Anspruch 1, weiterhin umfassend:
Öffnen einer Tür zu der Umgebungsfeuchtekammer (402) vor dem Laden des Klappenrahmens (102) und der Segelbildungsstruktur (202); und
Schließen der Tür zu der Umgebungsfeuchtekammer (402) nach dem Laden des Klappenrahmens (102) und der Segelbildungsstruktur (202).

6. Verfahren nach Anspruch 1, wobei das automatische Bewegen des Klappenrahmens (102) und der Segelbildungsstruktur (202) durch die Umgebungsfeuchtekammer ein Bewegen des Klappenrahmens (102) und der Segelbildungsstruktur (202) durch mindestens 50 % einer Querschnittsfläche der Umgebungsfeuchtekammer (402) umfasst.

7. Verfahren nach Anspruch 1, wobei kein Gas innerhalb der Umgebungsfeuchtekammer (402) während dem Zeitraum von einem Gebläse angetrieben wird.

8. Verfahren nach Anspruch 1, weiterhin umfassend ein Herausnehmen des Klappenrahmens (102) und der Segelbildungsstruktur (202) aus der Umgebungsfeuchtekammer (402) .

9. Verfahren nach Anspruch 1, weiterhin umfassend ein Abschneiden von überschüssigem zumindest zum Teil ausgehärtetem Polymer von dem Klappenrahmen (102).

10. Verfahren nach Anspruch 1, wobei, wenn die Umgebungsfeuchtekammer (402) geschlossen ist und der Klappenrahmen (102) automatisch durch die Umgebungsfeuchtekammer (402) bewegt wird, und wobei die Feuchte in der Umgebungsfeuchtekammer (402) auf ± 1 % innerhalb der Umgebung mit kontrollierter Feuchte von mehr als 50 % und weniger als 95 % kontrolliert wird.

11. Verfahren nach Anspruch 1, wobei die Umgebungsfeuchtekammer (402) ein Volumen von 16.387,1 Kubikzentimeter (1000 Kubikzoll) oder weniger aufweist.

12. Verfahren nach Anspruch 1, wobei der Klappenrahmen (102) eine maschinenlesbare optische Kennung darauf aufweist.

13. Verfahren nach Anspruch 1, weiterhin umfassend ein Zuschneiden von ausgehärtetem Polymer, um eine Vielzahl von Segeln (110) auf dem Klappenrahmen (102) zu bilden.

14. Verfahren nach Anspruch 1, wobei das Polymer, das eine Vielzahl von Segeln (110) auf der Klappenstruktur bilden wird, nach dem Herausnehmen aus der zweiten Kammer (403) zwischen 70 und 200 Mikron dick ist.

15. Verfahren nach Anspruch 1, wobei der Klappenrahmen (102) ein erster Klappenrahmen (102) ist und die Segelbildungsstruktur (202) eine erste Segelbildungsstruktur (202) ist, wobei das Verfahren weiterhin umfasst:
Aufbringen eines Polymers im flüssigen Zustand auf einen zweiten Klappenrahmen (102) und eine zweite Segelbildungsstruktur (202);
Laden des zweiten Klappenrahmens (102) und der zweiten Segelbildungsstruktur (202) mit dem Polymer im flüssigen Zustand in die Umgebungsfeuchtekammer (402); und
automatisches Bewegen des ersten Klappenrahmens (102), der ersten Segelbildungsstruktur (202), des zweiten Klappenrahmens (102) und der zweiten Segelbildungsstruktur (202) durch die Umgebungsfeuchtekammer (402) für den Zeitraum, während dem das Polymer auf dem ersten Klappenrahmen (102), der ersten Segelbildungsstruktur (202), dem zweiten Klappenrahmen (102) und der zweiten Segelbildungsstruktur (202) zumindest zum Teil aushärtet;
wobei das Verfahren optional weiterhin umfasst:
Herausnehmen des zweiten Klappenrahmens (102) und der zweiten Segelbildungsstruktur (202) aus der Umgebungsfeuchtekammer (402) nach dem Zeitraum, wobei das Polymer nur zum Teil ausgehärtet ist;
Einbringen des zweiten Klappenrahmens (102) und der zweiten Segelbildungsstruktur (202) in eine zweite Umgebungsfeuchtekammer (403);
Erhitzen des zweiten Klappenrahmens (102) und der zweiten Segelbildungsstruktur (202) in der zweiten Umgebungsfeuchtekammer (403), bis das Polymer aushärtet; und
Zuschneiden von ausgehärtetem Polymer, um eine Vielzahl von Segeln (110) auf dem zweiten Klappenrahmen (102) zu bilden; optional wobei
eine Variation der Dicke zwischen jedem von der Vielzahl von Segeln (110) auf dem ersten Klappenrahmen (102) und jedem von der Vielzahl von Segeln (110) auf dem zweiten Klappenrahmen eine Dicke von weniger als 10 Mikron aufweist.

## Revendications

1. Procédé de fabrication de feuillets valvulaires (110), comprenant :
l'application d'un polymère à l'état liquide sur une armature de valve (102) et une structure de formation de feuillets (202) par trempage de l'armature de valve (102) en immergeant au moins une partie de l'armature de valve (102) et de la structure de formation de feuillets (202) dans un réservoir rempli du polymère à l'état liquide ;
un chargement de l'armature de valve (102) et de la structure de formation de feuillets (202), avec le polymère à l'état liquide, dans une première chambre à humidité ambiante (402) pourvue d'un capteur d'humidité (504) et d'un équipement de contrôle (405) pour maintenir un environnement à humidité contrôlée supérieure à 50 % et inférieure à 95 % ;
un déplacement automatique de l'armature de valve (102) et de la structure de formation de feuillets (202) autour de la chambre à humidité ambiante (402) pendant une période de temps durant laquelle l'humidité à l'intérieur de la chambre à humidité ambiante (402) est contrôlée et durant laquelle le polymère durcit au moins en partie ; le procédé comprenant en outre :
un retrait de l'armature de valve (102) et de la structure de formation de feuillets (202) de la première chambre à humidité ambiante (402) une fois que la période de temps s'est écoulée, le polymère étant seulement en partie durci ;
une insertion de l'armature de valve (102) et de la structure de formation de feuillets (202) dans une deuxième chambre à humidité ambiante (403) ; et
un chauffage de l'armature de valve (102) et de la structure de formation de feuillets (202) dans la deuxième chambre à humidité ambiante (403) jusqu'à ce que le polymère durcisse.

2. Procédé selon la revendication 1, dans lequel le déplacement automatique de l'armature de valve (102) et de la structure de formation de feuillets (202) autour de la chambre à humidité ambiante (402) pendant la période de temps durant laquelle le polymère durcit au moins en partie comprend :
une rotation automatique de l'armature de valve (102) et de la structure de formation de feuillets (202) autour de la chambre à humidité ambiante (402) dans un plan perpendiculaire à un axe.

3. Procédé selon la revendication 2, dans lequel l'armature de valve (102) et la structure de formation de feuillets (202) sont tournées sur au moins un tour complet autour de l'axe.

4. Procédé selon la revendication 1, dans lequel l'environnement à humidité contrôlée de la chambre à humidité ambiante (402) est fonctionnellement lié à un milieu humide utilisé comme source d'humidité.

5. Procédé selon la revendication 1, comprenant en outre :
l'ouverture d'une porte vers la chambre à humidité ambiante (402) avant le chargement de l'armature de valve (102) et de la structure de formation de feuillets (202) ; et
la fermeture de la porte de la chambre à humidité ambiante (402) après le chargement de l'armature de valve (102) et de la structure de formation de feuillets (202).

6. Procédé selon la revendication 1, dans lequel le déplacement automatique de l'armature de valve (102) et de la structure de formation de feuillets (202) autour de la chambre à humidité ambiante comprend un déplacement de l'armature de valve (102) et de la structure de formation de feuillets (202) à travers au moins 50 % d'une section transversale de la chambre à humidité ambiante (402).

7. Procédé selon la revendication 1, dans lequel aucun gaz n'est entraîné par un ventilateur à l'intérieur de la chambre à humidité ambiante (402) pendant la période de temps.

8. Procédé selon la revendication 1, comprenant en outre un retrait de l'armature de valve (102) et de la structure de formation de feuillets (202) de la chambre à humidité ambiante (402).

9. Procédé selon la revendication 1, comprenant en outre un découpage du polymère au moins en partie durci excédentaire de l'armature de valve (102).

10. Procédé selon la revendication 1, dans lequel, lorsque la chambre à humidité ambiante (402) est fermée et l'armature de valve (102) est déplacée automatiquement autour de la chambre à humidité ambiante (402), et dans lequel l'humidité dans la chambre à humidité ambiante (402) est contrôlée à ± 1 % dans l'environnement à humidité contrôlée de façon à être supérieure à 50 % et inférieure à 95 %.

11. Procédé selon la revendication 1, dans lequel la chambre à humidité ambiante (402) a un volume de 16 387,1 centimètres cubes (1 000 pouces cubes) ou moins.

12. Procédé selon la revendication 1, dans lequel l'armature de valve (102) porte un identificateur optique lisible par machine.

13. Procédé selon la revendication 1, comprenant en outre un découpage du polymère durci pour former une pluralité de feuillets (110) sur l'armature de valve (102).

14. Procédé selon la revendication 1, dans lequel, après avoir été retiré de la deuxième chambre (403), le polymère destiné à former une pluralité de feuillets (110) sur la structure de valve a une épaisseur comprise entre 70 et 200 microns.

15. Procédé selon la revendication 1, dans lequel l'armature de valve (102) est une première armature de valve (102) et la structure de formation de feuillets (202) est une première structure de formation de feuillets (202), le procédé comprenant en outre :
l'application d'un polymère à l'état liquide sur une deuxième armature de valve (102) et une deuxième structure de formation de feuillets (202) ;
le chargement de la deuxième armature de valve (102) et de la deuxième structure de formation de feuillets (202), avec le polymère à l'état liquide, dans la chambre à humidité ambiante (402) ; et
un déplacement automatique de la première armature de valve (102), de la première structure de formation de feuillets (202), de la deuxième armature de valve (102) et de la deuxième structure de formation de feuillets (202) autour de la chambre à humidité ambiante (402) pendant la période de temps durant laquelle le polymère sur la première armature de valve (102), la première structure de formation de feuillets (202), la deuxième armature de valve (102) et la deuxième structure de formation de feuillets, (202) durcit au moins en partie ; le procédé comprenant optionnellement en outre :
un retrait de la deuxième armature de valve (102) et de la deuxième structure de formation de feuillets (202) de la chambre à humidité ambiante (402) une fois que la période de temps s'est écoulée, le polymère étant seulement en partie durci ;
une insertion de la deuxième armature de valve (102) et de la deuxième structure de formation de feuillets (202) dans une deuxième chambre à humidité ambiante (403) ;
un chauffage de la deuxième armature de valve (102) et de la deuxième structure de formation de feuillets (202) dans la deuxième chambre à humidité ambiante (403) jusqu'à ce que le polymère durcisse ; et
un découpage du polymère durci pour former une pluralité de feuillets (110) sur la deuxième armature de valve (102) ; optionnellement dans lequel
la variation d'épaisseur entre chacun de la pluralité de feuillets (110) sur la première armature de valve (102) et chacun de la pluralité de feuillets (110) sur la deuxième armature de valve est inférieure à 10 microns d'épaisseur.
